# EUROPÄISCHE PATENTSCHRIFT

(11) **EP 1 617 792 B1**
(45) Veröffentlichungstag und Bekanntmachung des Hinweises auf die Patenterteilung: **18.11.2009**
(21) Anmeldenummer: 03709539.5
(22) Anmeldetag: 10.04.2003
(51) Int. Cl.: A61F 2/42, A61B 17/86, A61B 17/84

(54) **VORRICHTUNG ZUR TEMPORÄREN SCHIENUNG VON ZEHEN**
DEVICE FOR THE TEMPORARY SPLINTING OF TOES
DISPOSITIF POUR ECLISSER TEMPORAIREMENT DES ORTEILS

(43) Veröffentlichungstag der Anmeldung: 25.01.2006
(73) Patentinhaber: Synthes GmbH, 4436 Oberdorf (CH)
(72) Erfinder: KLAUE, Kaj, CH-6942 Savosa (CH); ZWIPP, Hans, 01326 Dresden (DE); CRONIER, Patrick, F-49000 Angers (FR); SANDS, Andrew, K., Amagansett, NY 11930-7136 (US)
(74) Vertreter: Lusuardi, Werther
(86) Internationale Anmeldenummer: PCT/CH2003/000236
(87) Internationale Veröffentlichungsnummer: WO 2004/089255

(56) Entgegenhaltungen:
- EP-A- 0 682 917
- FR-A- 2 809 300
- GB-A- 2 355 505
- US-A- 4 723 541
- US-A- 5 871 486
- US-A- 6 056 749

## Beschreibung

Die Erfindung bezieht sich auf eine Vorrichtung gemäss dem Oberbegriff des Patentanspruchs 1.

Solche Vorrichtungen dienen der temporären Schienung von Zehen, insbesondere zur Behandlung von Hammerzehen oder anderen Fehlstellungen von Zehen.

Stand der Technik für diese Indikationen ist die Kirschnerdraht-Fixation der Zehengelenke während der Heilungszeit (Weichteil- und/oder Knochenheilung) wobei der Draht an der Zehenspitze hervorragt. Der Nachteil dieser bekannten Technik besteht darin, dass der Patient kaum arbeitsfähig ist, da er eine gewisse "Stosstange" (z.B. eine harte Schiene) tragen muss.
Die häufigste Operation dieser Art ist die Arthrodese des proximalen Interphalangeal-Gelenkes, d.h. das Zusammenwachsen der Knochen, wobei leider oft nur die Gelenkresektion durchgeführt wird (sogenannte Operation nach Hohmann). Es wird auch die funktionelle Operation empfohlen, wobei Sehnen der Endphalanx auf die Grundphalanx transferiert werden (sogenannte Operation nach Girdlestone und Taylor 1947). Beide Operationen benötigen 6 bis 8 Wochen mechanische Ruhigstellung.

Aus der US 5,207,712 ist ein absorbierbares Implantat zur Korrektur von Fehlstellungen von Zehen bekannt. Nachteilig bei dieser bekannten Vorrichtung ist ihre geradlinige Form und die fehlende Kannulierung, so dass die Verwendung eines Führungsdrahtes unmöglich ist. Ein weiterer Nachteil liegt darin begründet, dass das Implantat gemäss der US 5,207,712 eine grosse Knochenentfernung notwendig macht.

FR-A-2 809 300 offenbart eine Vorrichtung nach dem Oberbegriff des Anspruchs 1.

Hier will die Erfindung Abhilfe schaffen. Der Erfindung liegt die Aufgabe zugrunde, eine Vorrichtung zu schaffen, mit der es möglich ist, den Bodenkontakt der Zehenbeere wiederherzustellen.

Die Erfindung löst die gestellte Aufgabe mit einer Vorrichtung, welche die Merkmale des Anspruchs 1 aufweist.

Die durch die Erfindung erreichten Vorteile sind im wesentlichen darin zu sehen, dass dank der erfindungsgemässen Vorrichtung:
a) eine postoperative Entfernung des implantierten bioresorbieren Rohres entfällt;
b) die 6-bis 8-wöchige "Invalidität" des Patienten wegen der aus den Zehen hervortretenden Drähte entfällt; und
c) transfixierte Gelenke, die nicht definitiv zusammenwachsen sollen, können geschlossen nach 6 bis 8 Wochen "arthrolysiert" werden. Dabei wird das bioresorbierbare Rohr vom Arzt gezielt gebrochen.

In einer bevorzugten Ausführungsform weist das Rohr eine in einer Ebene liegende Biegung auf. Der Vorteil dieser Ausgestaltung liegt im wesentlichen darin, dass während der Heilungszeit dadurch der Bodenkontakt der Zehenbeere leicht federnd wird, d.h. mit einer leichten "Vorspannung" in Beugung gebracht wird.

In einer anderen Ausführungsform ist die Biegung des Rohres S-förmig ausgestaltet, wodurch eine anatomischen vorteilhaften Krümmung der zu korrigierenden Zehe erreichbar ist.

Bei einer weiteren Ausführungsform ist das Rohr mindestens an einem seiner Enden verjüngt. Vorzugsweise läuft das verjünge Ende in eine Spitze aus. Damit kann das Rohr leichter implantiert werden.

In wiederum einer anderen Ausführungsform ist der Führungsdraht geradlinig, so dass dieser mit dem Bohrer in das Rohr eingetrieben werden kann.

In einer weiteren Ausführungsform weist das bioresorbierbare Material eine Bruchdehnung ε = (ΔI x 100/L) < 10 % auf. Der Vorteil eines solchen Material liegt in seiner besseren Resorbierbarkeit. Vorzugsweise beträgt die Länge des Implantates insitu ca. 5 cm.

Als bioresorbierbare Materialien eignen sich insbesondere L-Polylactid oder Caprolacton. Diese Materialien weisen den Vorteil auf, dass sie schneller via Gelenkflüssigkeit resorbieren.

Die Aussenfläche des Rohres ist glatt ausgestaltet.

Die Erfindung und Weiterbildungen der Erfindung werden im folgenden anhand einer teilweise schematischen Darstellungen eines Ausführungsbeispiels noch näher erläutert.

Es zeigen:
Fig. 1 eine Aufsicht auf eine Zehe mit endomedullär eingeführtem Führungsdraht und bioresorbierbarem Rohr;
Fig. 2 einen Querschnitt durch die erfindungsgemässe Vorrichtung nach Fig. 1;
Fig. 3 einen seitliche Ansicht durch die Zehe nach Fig. 1 mit dem endomedullär eingeführten bioresorbierbaren Rohr nach erfolgter Entfernung des Führungsdrahtes; und
Fig. 4 eine Aufsicht auf eine Zehe mit endomedullär eingeführtem Führungsdraht und einem anderen nicht erfindungsgemäßen bioresorbierbaren Rohr.

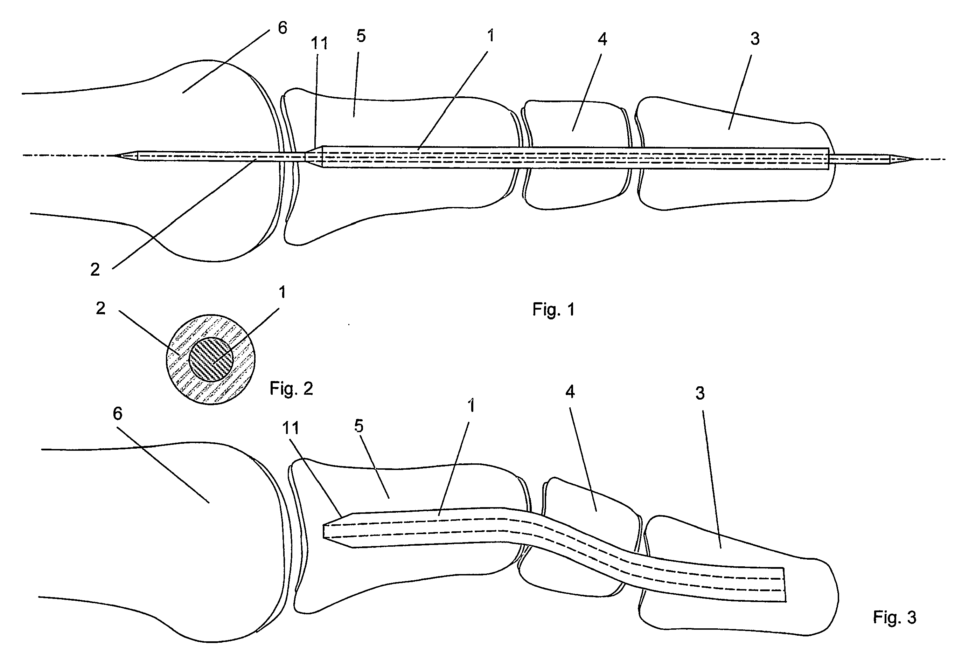

Die in den Fig. 1 bis 3 dargestellte Vorrichtung zur temporären Schienung von Zehen, insbesondere zur Behandlung von Hammerzehen oder anderen Fehlstellungen von Zehen besteht im wesentlichen aus einem Rohr 1 mit einem Innendurchmesser d, das aus einem bioresorbierbaren Material (L-Polylactid) besteht sowie einem Führungsdraht 2 aus Metall, welcher einen Aussendurchmesser D ≤ d besitzt. Das Rohr 1 weist eine in der Zeichenebene liegende Biegung in Form eines S auf. Der Innendurchmesser d des Rohres 1 beträgt 1,15 mm, seine Wandstärke 0,25 mm und die Länge des Rohres 1 beträgt 4,5 cm. Die Oberfläche des Rohres 1 ist völlig glatt. Das eine Ende des Rohres 1, welches zur Einführung in die Zehe bestimmt ist, ist verjüngt ausgebildet, so dass es in eine Spitze 11 ausläuft.

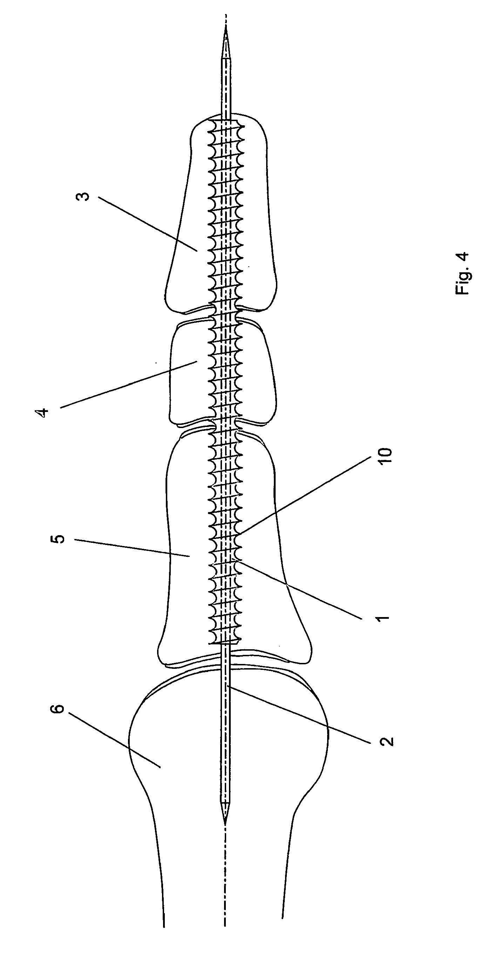

In Fig. 4 ist ein Rohr 1 dargestellt, welche sich von der in Fig. 1 dargestellten Ausführungsform nur darin unterscheidet, dass die Oberfläche des Rohres 1 mit einem Aussengewinde 10 versehen ist.

Zum besseren Verständnis der erfindungsgemässen Vorrichtung folgt ein kurzer Operationsbericht:
Der chirurgische Zugang erfolgt auf der Mittellinie des Zehenrückens. Das proximale Interphalangeal-Gelenk wird arthrotomiert und die Gelenksflächen dargestellt. Der gesamt Knorpel wird nun entfernt und die Basis der Mittelphalanx mit einem 3,5 mm Bohrer perforiert. Die Grundphalanx wird nun spitzig zugeschnitten. Die damit leicht verkürzte Zehe kann von ihrer rigiden Hammerzehen-Deformation aufgerichtet werden und die Grundphalanx wird in die Mittelphalanx zur Probe eingesteckt.
Nun wird ein Führungsdraht aus Metall durch das 3,5 mm Loch axial durch die gesamte Zehe nach distal durch die Zehenbeere ausgedreht.
Der Bohrer wird nun am distal herausragenden Führungsdraht befestigt, worauf der Führungsdraht weiter herausgezogen wird, bis seine Spitze an der Basis der Mittelphalanx gerade noch sichtbar ist.
Die Grundphalanx wird nun definitiv in die Mittelphalanx gesteckt und mit einer korrekten Metatarsophalangeal-Gelenkstellung mit der Hand gehalten.
Jetzt wird der Führungsdraht mit dem Bohrer nach proximal durch das richtig stehende Mefiafiarsophalangeal-Gelenk, bis in den Metatarsus gedreht. In selteneren Fällen kann der Führungsdraht auch bis zur Basis der Grundphalanx gedreht werden.
Der resorbierbare Kanülendraht bzw. das Implantat wird nun über den Führungsdraht bis zur gewünschten Stelle eingedreht.
Kurz davor wird das Implantat an der Zehenbeere mit einer Spezialbeisszange abgeschnitten.
Mit einem Rohrstössel wird das Rohr nun über dem Führungsdraht einige Millimeter unter die Haut und bis zur Knochenspitze der Endphalanx eingeschlagen.
Mit manueller Sicherung des Rohrstössels wird der Führungsdraht schliesslich nach distal mit der Bohrmaschine herausgedreht.

In einer anderen Anwendungstechnik wird beim Operationsschritt I) der Führungsdraht 2 und folglich das Rohr 1 bis in das Metatarsale-Köpfchen eingeführt, was insbesondere im Falle einer metatarso-phalangealen Instabilität vorteilhaft ist.

In einer weiteren Anwendungstechnik wird 6 bis 8 Wochen nach erfolgter Implantation des Rohres 1, dieses vom Arzt manuell auf Höhe des Metatarsophalangeal-Gelenkes gebrochen um die Gelenkigkeit der Zehe wiederherzustellen.

## Patentansprüche

1. Vorrichtung zur temporären Schienung von Zehen, insbesondere zur Behandlung von Hammerzehen oder anderen Fehlstellungen von Zehen bestehend aus einem Rohr (1) mit einem Innendurchmesser d und einem Führungsdraht (2), welcher einen Aussendurchmesser D ≤ d besitzt,
**dadurch gekennzeichnet, dass**
A) das Rohr (1) aus einem bioresorbierbaren Material besteht; und
B) das Rohr (1) eine glatte Aussenfläche aufweist.

2. Vorrichtung nach Anspruch 1, **dadurch gekennzeichnet, dass** das Rohr (1) mindestens an einem seiner Enden verjüngt ist.

3. Vorrichtung nach Anspruch 2, **dadurch gekennzeichnet, dass** das Rohr (1) mindestens an einem seiner Enden in eine Spitze (11) ausläuft.

4. Vorrichtung nach einem der Ansprüche 1 bis 3, **dadurch gekennzeichnet, dass** der Führungsdraht (2) mindestens an einem seiner Enden in eine Spitze ausläuft.

5. Vorrichtung nach einem der Ansprüche 1 bis 4, **dadurch gekennzeichnet, dass** das Rohr (1) eine in einer Ebene liegende Biegung, vorzugsweise eine S-förmige Biegung aufweist.

6. Vorrichtung nach einem der Ansprüche 1 bis 5, **dadurch gekennzeichnet, dass** der Führungsdraht (2) geradlinig ist.

7. Vorrichtung nach einem der Ansprüche 1 bis 6, **dadurch gekennzeichnet, dass** das bioresorbierbare Material im wesentlichen spröde und brüchig ist.

8. Vorrichtung nach einem der Ansprüche 1 bis 7, **dadurch gekennzeichnet, dass** das bioresorbierbare Material eine Bruchdehnung ε = (ΔI x 100/L) < 10 % aufweist.

9. Vorrichtung nach einem der Ansprüche 1 bis 8, **dadurch gekennzeichnet, dass** das bioresorbierbare Material ein L-Polylactid oder eine Caprolacton ist.

10. Vorrichtung nach einem der Ansprüche 1 bis 9, **dadurch gekennzeichnet, dass** der Innendurchmesser d des Rohres (1) grösser als 0,5 mm, vorzugsweise grösser als 1, 0 mm ist.

11. Vorrichtung nach einem der Ansprüche 1 bis 10, **dadurch gekennzeichnet, dass** der Innendurchmesser d des Rohres (1) kleiner als 1,60 mm, vorzugsweise kleiner als 1,3 mm ist.

12. Vorrichtung nach einem der Ansprüche 1 bis 11, **dadurch gekennzeichnet, dass** die Wandstärke des Rohres (1) grösser als 0,1 mm, vorzugsweise grösser als 0,15 mm ist.

13. Vorrichtung nach einem der Ansprüche 1 bis 12, **dadurch gekennzeichnet, dass** die Wandstärke des Rohres (1) kleiner als 0,5 mm, vorzugsweise kleiner als 0,4 mm ist.

14. Vorrichtung nach einem der Ansprüche 1 bis 13, **dadurch gekennzeichnet, dass** die Länge des Rohres (1) 3 - 6 cm, vorzugsweise 4 - 5 cm ist.

## Claims

1. Device for the temporary splinting of toes, in particular for treating hammer toes or other toe defects, consisting of a tube (1) with an inner diameter d and a guiding wire (2) having an outer diameter D ≤ d,
**characterized in that**
(A) the tube (1) consists of a bio-absorbing material; and
(B) the tube (1) has a smooth outer surface.

2. Device according to claim 1, **characterized in that** the tube (1) is tapered on at least one of its ends.

3. Device according to claim 2, **characterized in that** the tube (1) tapers off to a tip (11) on at least one of its ends.

4. Device according to one of the claims from 1 to 3, **characterized in that** the guiding wire (2) tapers off to a tip on at least one of its ends.

5. Device according to one of the claims from 1 to 4, **characterized in that** the tube (1) presents a bend lying in a plane, preferably an S-shaped bend.

6. Device according to one of the claims from 1 to 5, **characterized in that** the guiding wire (2) is straight.

7. Device according to one of the claims from 1 to 6, **characterized in that** the bio-absorbing material is essentially brittle and friable.

8. Device according to one of the claims from 1 to 7, **characterized in that** the bio-absorbing material has a breaking elongation ε = (ΔI x 100/L) < 10%.

9. Device according to one of the claims from 1 to 8, **characterized in that** the bio-absorbing material is an L-polyactide or a caprolactone.

10. Device according to one of the claims from 1 to 9, **characterized in that** the inner diameter of the tube (1) is greater than 0.5 mm, preferably greater than 1.0 mm.

11. Device according to one of the claims from 1 to 10, **characterized in that** the inner diameter of the tube (1) is smaller than 1.60 mm, preferably smaller than 1.3 mm.

12. Device according to one of the claims from 1 to 11, **characterized in that** the wall thickness of the tube (1) is greater than 0.1 mm, preferably greater than 0.15 mm.

13. Device according to one of the claims from 1 to 12, **characterized in that** the thickness of the tube (1) is smaller than 0.5 mm, preferably smaller than 0.4 mm.

14. Device according to one of the claims from 1 to 13, **characterized in that** the length of the tube (1) is 3 - 6 cm, preferably 4 - 5 cm.

## Revendications

1. Dispositif pour la contention temporaire d'orteils, en particulier pour le traitement d'orteils en marteau ou d'autres déformations des orteils, composé d'un tube (1) présentant un diamètre intérieur d et d'un fil-guide (2), lequel fil-guide présente un diamètre extérieur D ≤ d,
**caractérisé en ce que**
A) le tube (1) consiste en un matériau biorésorbable ; et
B) le tube (1) présente une surface externe lisse.

2. Dispositif selon la revendication 1, **caractérisé en ce que** le tube (1) est effilé, au moins au niveau de l'une de ses extrémités.

3. Dispositif selon la revendication 2, **caractérisé en ce que** le tube (1) se termine par une pointe (11), au moins au niveau de l'une de ses extrémités.

4. Dispositif selon l'une quelconque des revendications 1 à 3, **caractérisé en ce que** le fil-guide (2) se termine par une pointe, au moins au niveau de l'une de ses extrémités.

5. Dispositif selon l'une quelconque des revendications 1 à 4, **caractérisé en ce que** le tube (1) présente une courbure s'étendant dans un plan, de préférence une courbure en forme de S.

6. Dispositif selon l'une quelconque des revendications 1 à 5, **caractérisé en ce que** le fil-guide (2) est rectiligne.

7. Dispositif selon l'une quelconque des revendications 1 à 6, **caractérisé en ce que** le matériau biorésorbable est essentiellement fragile et cassant.

8. Dispositif selon l'une quelconque des revendications 1 à 7, **caractérisé en ce que** le matériau biorésorbable présente un allongement à la rupture ε = (ΔI x 100/L) < 10%.

9. Dispositif selon l'une quelconque des revendications 1 à 8, **caractérisé en ce que** le matériau biorésorbable est un L-polylactide ou une caprolactone.

10. Dispositif selon l'une quelconque des revendications 1 à 9, **caractérisé en ce que** le diamètre intérieur d du tube (1) est supérieur à 0,5 mm, de préférence supérieur à 1,0 mm.

11. Dispositif selon l'une quelconque des revendications 1 à 10, **caractérisé en ce que** le diamètre intérieur d du tube (1) est inférieur à 1,60 mm, de préférence inférieur à 1,3 mm.

12. Dispositif selon l'une quelconque des revendications 1 à 11, **caractérisé en ce que** l'épaisseur de paroi du tube (1) est supérieure à 0,1 mm, de préférence supérieure à 0,15 mm.

13. Dispositif selon l'une quelconque des revendications 1 à 12, **caractérisé en ce que** l'épaisseur de paroi du tube (1) est inférieure à 0,5 mm, de préférence inférieure à 0,4 mm.

14. Dispositif selon l'une quelconque des revendications 1 à 13, **caractérisé en ce que** la longueur du tube (1) est de 3 à 6 cm, de préférence de 4 à 5 cm.
